# EUROPÄISCHE PATENTSCHRIFT

(11) **EP 3 683 200 B1**
(45) Veröffentlichungstag und Bekanntmachung des Hinweises auf die Patenterteilung: **27.11.2024**
(21) Anmeldenummer: 19020033.7
(22) Anmeldetag: 18.01.2019
(51) Int. Cl.: C07C 1/20, C07C 11/06, C10G 3/00, C07C 4/06, C07C 7/00, C07C 7/09, C07C 11/02

(54) **VERFAHREN ZUR HERSTELLUNG VON OLEFINEN AUS METHANOL UND/ODER DIMETHYLETHER**
PROCESS FOR PRODUCING OLEFINS FROM METHANOL AND/OR DIMETHYL ETHER
PROCÉDÉ DE PRODUCTION D'OLÉFINES À PARTIR DE MÉTHANOL ET/OU D'ÉTHER DIMÉTHYLIQUE

(43) Veröffentlichungstag der Anmeldung: 22.07.2020
(73) Patentinhaber: L'AIR LIQUIDE, SOCIÉTÉ ANONYME POUR L'ÉTUDE ET L'EXPLOITATION DES PROCÉDÉS GEORGES CLAUDE, 75007 Paris (FR)
(72) Erfinder: Ahlers, Bernd, 63128 Dietzenbach (DE); Castillo-Welter, Frank, 61381 Friedrichsdorf (DE); Drosdzol, Christopher, 60438 Frankfurt (DE); Gorny, Martin, 65760 Eschborn (DE); Haag, Stéphane, 60598 Frankfurt (DE); Janko, Lutz, 64390 Erzhausen (DE); Williams, Bryce, 60437 Frankfurt am Main (DE)
(74) Vertreter: Air Liquide

(56) Entgegenhaltungen:
- WO-A1-2012/016786
- DE-A1- 102013 101 575
- DE-A1- 102014 118 967

## Beschreibung

### Gebiet der Erfindung

Die Erfindung betrifft ein Verfahren zur Herstellung von Olefinen aus Oxygenaten, umfassend folgende Schritte:
(i) Heterogen-katalysierte Umsetzung der Oxygenate in einem Oxygenat-zu-Olefin-Reaktor unter Oxygenatumwandlungsbedingungen zu einem Produktstrom, der Wasser, Kohlenwasserstoffe und wenigstens ein Oxygenat enthält,
(ii) Quenchen des Produktstroms in wenigstens einer Quenchstufe, wodurch ein gasförmige Kohlenwasserstoffe und Olefine umfassender Strom G, ein flüssiger, zum überwiegenden Teil aus Wasser und Oxygenaten bestehender wässriger Strom W und ein flüssiger, zum überwiegenden Teil aus Kohlenwasserstoffen bestehender und Olefine umfassender Strom O erhalten wird,
(iii) Auftrennen des Stroms G und/oder des Stroms O in einen Strom C4-, welcher zum überwiegenden Teil aus Kohlenwasserstoffen mit vier oder weniger Kohlenstoffatomen besteht und Olefine umfasst, und einen Strom C4+, welcher zum überwiegenden Teil aus Kohlenwasserstoffen mit vier oder mehr Kohlenstoffatomen besteht und Olefine umfasst.

Weiterhin umfasst die Erfindung auch die Verwendung einer korrespondierenden Anlage in dem erfindungsgemässen Verfahren.

### Stand der Technik

Kurzkettige Olefine, beispielsweise Propylen (Propen), gehören zu den wichtigsten Grundstoffen der chemischen Industrie. Dies liegt darin begründet, das ausgehend von diesen ungesättigten Verbindungen mit einer kurzen Kettenlänge Moleküle mit langkettigem Kohlenstoffgerüst und zusätzlichen Funktionalisierungen aufgebaut werden können.

Als Quelle für kurzkettige Olefine diente in der Vergangenheit vor allem das Steam-Cracking, d. h. die thermische Spaltung bei der Erdölverarbeitung. In den vergangenen Jahren wurden jedoch weitere Prozesse zur Herstellung von kurzkettigen Olefinen entwickelt. Dies ist zum einen durch steigende Nachfrage bedingt, die durch die vorhandenen Quellen nicht mehr gedeckt werden kann; zum anderen fordert die zunehmende Verknappung von fossilen Rohstoffen die Verwendung anderer Ausgangsstoffe.

Die sog. MTP- (Methanol-to-Propylen) oder auch MTO- (Methanol-to-Olefin) Verfahren zur Herstellung von Propylen und anderen kurzkettigen Olefinen gehen von Methanol als Ausgangsstoff aus. Verallgemeinernd spricht man in diesem Zusammenhang auch von Oxygenat-zu-Olefin-(OTO)-Verfahren, da sauerstoffhaltige organische Komponenten wie Methanol oder Dimethylether (DME) auch als Oxygenate bezeichnet werden. In diesen heterogen katalysierten Verfahren wird demnach beispielsweise aus Methanol zunächst teilweise das Zwischenprodukt Dimethylether und nachfolgend aus einer Mischung von Methanol und Dimethylether eine Mischung aus Ethylen und Propylen sowie Kohlenwasserstoffen mit höherer Molmasse, darunter auch Olefine, gebildet. Zudem findet sich im Produktstrom Wasser, welches einerseits einen aus dem Prozessdampf stammt, der optional dem MTO-Reaktor zur Reaktionsmodulierung zugeführt wird und andererseits aus dem im MTP-Reaktor erzeugten Reaktionswasser.

Die sich anschließende Aufreinigung soll zum einen unerwünschte Nebenprodukte und unumgesetzte Edukte abtrennen und die einzelnen Kohlenwasserstofffraktionen möglichst rein darstellen. Üblicherweise wird hierzu im ersten Schritt ein Quenchsystem zur Anwendung gebracht. Unter Quenchen wird dabei eine schlagartige oder schockartige Abkühlung verstanden, die zumeist durch direkten Wärmeaustausch mit einem fluiden Quenchmedium bewirkt wird. Wenn hierzu eine Flüssigkeit wie Wasser oder Methanol verwendet wird, tritt zudem eine gewisse Reinigungswirkung in Bezug auf die verbliebene Gasphase auf.

Ein Beispiel für die einer OTO-Reaktion nachfolgende Aufreinigung findet sich in der DE 10 2014 112 792 A1, in der beschrieben wird, wie in einem ersten Schritt eine heterogen-katalysierte Umsetzung von wenigstens einem Oxygenat zu einem C2 Olefine, C3 Olefine, C4 Olefine, C5/6 Kohlenwasserstoffverbindungen und C7+ Kohlenwasserstoffverbindungen enthaltenden Produktstrom und in einem zweiten Schritt eine Abtrennung eines zu wenigstens 95 Gew.-% aus C3 Olefinen bestehenden Propylenstroms erzeugt wird.

Die weiteren Aufreinigungseinheiten, die in der DE 10 2014 112 792 A1 beschrieben werden, entsprechen dem im Stand der Technik üblichen Konzept. Durch das Quenchen kann bereits eine grobe Trennung der Fraktionen in Abhängigkeit von ihrer Kettenlänge der entstehenden Olefine aufgrund des teilweisen Auskondensierens erfolgen, so dass eine flüssige C4+ Fraktion aus dem Quench abgeführt werden kann. Die gasförmig abgetrennte C4- Fraktion wird anschließend in eine Kompressionsstufe eingespeist. Die aus der Kompression stammende C4- Fraktion wird dann einer Trennvorrichtung zugeführt, in der C3- Kohlenwasserstoffe von den C4+ Kohlenwasserstoffen separiert werden. In anschließenden Reinigungsschritten wird die C3 Fraktion in einer weiteren Trenneinrichtung von der C2- Fraktion getrennt, was aufgrund der geringen Siedepunkte der beiden Fraktionen unter Druck erfolgen muss. Insgesamt ist die Aufreinigung des Produktstroms kompliziert, da eine hohe Reinheit der Produkte angestrebt wird. Dies gilt für MTP-Anlagen mit üblichen Produktionskapazitäten von etwa 470 kta Propylen, wird aber noch entscheidender, wenn die Anlagenkapazität geringer ist (100 kta oder 200 kta Propylen). Dadurch sind kleine Anlagen zur Zeit wirtschaftlich nicht rentabel.

Zur Optimierung der Propylenausbeute können olefinhaltige Ströme aus dem Bereich der Aufreinigung zumindest teilweise in den Reaktionsteil zurückgeführt werden. Die Erzeugung von sehr spezifischen Recycleströmen wie C2, C4 und C5 / C6 Olefine erfordert nach dem Stand der Technik mehrere Trennschritte. Ein Recycle der C7+ Olefine gibt es bisher nicht. Hingegen lehrt die DE 10 2014 112 792 A1, dass zusätzlich auch wenigstens 10 Gew.-% des Propylenstroms aus der Aufreinigung in die heterogen-katalysierte Umsetzung zurückgeführt werden. Die Rückführung von Propylen ist jedoch problematisch, da es sich ja bei Propylen gerade um das gewünschte Wertprodukt handelt und so evtl. die Gesamtausbeute des Prozesses gesenkt wird.

Die optimierte Anpassung der verschiedenen Recycleströme ist jedoch auch unabhängig von der Frage der Propylenrückführung sehr komplex, da die Zusammensetzung der einzelnen Ströme sowie die Prozessbedingungen in Kombination mit der Katalysatorleistung und -verteilung in dem Reaktor zu berücksichtigen sind. Zudem führt jede weitere Rückführleitung und der damit verbundene Regel- und Steuerungsbedarf zu höheren Investitionskosten.

Die Patentveröffentlichung DE 102013101575 A1 beschreibt ein Verfahren zur Herstellung von Olefinen aus Oxygenaten, das die folgenden Schritte umfasst:
(i) heterogen-katalysierte Umsetzung von wenigstens einem Oxygenat zu einem flüssige und gasförmige organische Verbindungen und Wasser enthaltenden Gesamtstrom und
(ii) Auftrennung des Gesamtstroms in einer ersten Trenneinrichtung in eine zu wenigstens 90 Vol.-% die gasförmigen organischen Verbindungen des Gesamtstroms enthaltende Fraktion, in eine zu wenigstens 90 Gew. -% die flüssigen organischen Verbindungen des Gesamtstroms enthaltende Fraktion und in eine zu wenigstens 90 Gew. -% das Wasser des Gesamtstroms enthaltende Fraktion.

Die internationale Patentveröffentlichung WO 2012/016786 A1 offenbart ein Verfahren zur Umwandlung eines Alkoholgemisches (A), das etwa 20 Gew.-% bis 100% Isobutanol enthält, um im wesentlichen Propylen herzustellen, umfassend: a) Einführen eines Stroms in einen Reaktor (A), der das Gemisch (A), gemischt mit einem Strom (D I), der Olefine mit 4 Kohlenstoffatomen oder mehr (C4+-Olefine), gegebenenfalls Wasser, gegebenenfalls eine inerte b) Inkontaktbringen des Stroms mit einem Katalysator (AI) bei einer Temperatur über 500°C in dem Reaktor (A) unter Bedingungen, die wirksam sind mindestens einen Teil des Isobutanols und gegebenenfalls anderer Alkohole zu dehydratisieren und ein Cracken durchzuführen, c) Gewinnen aus dem Reaktor (A) einen Abfluss, umfassend: Ethylen, Propylen, Wasser, gegebenenfalls nicht umgesetzte Alkohole der Mischung (A), verschiedene Kohlenwasserstoffe, und die optionale inerte Komponente aus Schritt a), d) Fraktionieren des Abflusses aus Schritt c), um mindestens einen Ethylenstrom, einen Propylenstrom Strom, eine Fraktion, die im wesentlichen aus Kohlenwasserstoffen mit 4 oder mehr Kohlenstoffatomen besteht, Wasser und die optionale inerte Komponente des Schritts a) zu erzeugen, wobei gegebenenfalls Ethylen ganz oder teilweise am Einlass des Reaktors (A) rezykliert wird, wobei gegebenenfalls die Fraktion die im Wesentlichen aus Kohlenwasserstoffen mit 4 Kohlenstoffatomen oder mehr besteht, am Einlass des Reaktors (A) zurückgeführt wird.

Nachteilig ist es, dass die beiden zuletzt genannten Patentpublikationen keine spezifischen und überzeugenden Lösungen zur verbesserten und werterhöhenden Nutzung eines im Verfahren erhaltenen C4+ Stroms anbieten.

### Beschreibung der Erfindung

Es ist daher die Aufgabe der vorliegenden Erfindung, es neues Konzept für die Recycleströme bereitzustellen, durch welches erhebliche Einsparungen bei den Investitionskosten ohne Beeinträchtigung der Propylenausbeute erreicht werden können. Diese Aufgabe wird durch ein Verfahren mit den Merkmalen des Anspruchs 1, und durch eine Verwendung mit den Merkmalen des Anspruchs 10 gelöst. Weitere, insbesondere vorteilhafte Ausgestaltungen finden sich in den jeweiligen abhängigen Ansprüchen.

### Erfindungsgemäßes Verfahren:

Verfahren zur Herstellung von Olefinen aus Oxygenaten, umfassend die folgenden Schritte
(i) Heterogen-katalysierte Umsetzung der Oxygenate in einem Oxygenat-zu-Olefin-Reaktor unter Oxygenatumwandlungsbedingungen zu einem Produktstrom, der Wasser, Kohlenwasserstoffe und wenigstens ein Oxygenat enthält,
(ii) Quenchen des Produktstroms in wenigstens einer Quenchstufe, wodurch ein gasförmige Kohlenwasserstoffe und Olefine umfassender Strom G, ein flüssiger, zum überwiegenden Teil aus Wasser und Oxygenaten bestehen-der wässriger Strom W und ein flüssiger, zum überwiegenden Teil aus Kohlenwasserstoffen bestehender und Olefine umfassender Strom O erhalten wird,
(iii) Auftrennen des Stroms G und/oder des Stroms O in einen Strom C4-, welcher zum überwiegenden Teil aus Kohlenwasserstoffen mit vier oder weniger Kohlenstoffatomen besteht und Olefine umfasst, und einen Strom C4+, welcher zum überwiegenden Teil aus Kohlenwasserstoffen mit vier oder mehr Kohlenstoffatomen besteht und Olefine umfasst,
dadurch gekennzeichnet, dass der in Schritt (iii) erhaltene Strom C4+ direkt zu Schritt (i) zurückgeführt und/oder in eine Benzinfraktion geleitet wird.

Das Einleiten des in Schritt (iii) erhaltenen Stroms C4+ in eine Benzinfraktion kann bevorzugt durch Einleiten in eine Benzin-Stabilisatorkolonne erfolgen, die zur Abtrennung flüchtiger Bestandteile aus der Benzinfraktion durch Destillation oder Rektifikation dient, um beispielsweise die Lagerfähigkeit der Benzinfraktion zu verbessern.

Die für die Umsetzung von Oxygenaten zu Olefinprodukten erforderlichen Oxygenatumwandlungsbedingungen sind dem Fachmann aus dem Stand der Technik, beispielsweise den eingangs erörterten Druckschriften, bekannt. Es sind diejenigen physikalisch-chemischen Bedingungen, unter denen ein messbarer, bevorzugt ein technisch relevanter Umsatz von Oxygenaten zu Olefinen erzielt wird. Notwendige Anpassungen dieser Bedingungen an die jeweiligen Betriebserfordernisse wird er auf der Grundlage von Routineversuchen vornehmen. Dabei können ggf. offenbarte, spezifische Reaktionsbedingungen als Orientierung dienen, sie sind aber in Bezug auf den Umfang der Erfindung nicht einschränkend zu verstehen.

Thermische Trennverfahren im Sinne der Erfindung sind alle Trennverfahren, die auf der Einstellung eines thermodynamischen Phasengleichgewichtes beruhen. Bevorzugt sind die die Destillation oder Rektifikation. Grundsätzlich ist aber auch der Einsatz anderer thermischer Trennverfahren denkbar, beispielsweise der Extraktion oder Extraktivdestillation.

Als Aufreinigungsschritt sind im Zusammenhang mit der vorliegenden Erfindung grundsätzlich alle Verfahrensschritte zu betrachten, die sich eines thermischen Trennverfahrens bedienen; bevorzugt wird die Destillation oder Rektifikation verwendet.

Unter Fluidverbindung zwischen zwei Bereichen oder Anlagenteilen wird dabei jegliche Art von Verbindung verstanden, die es ermöglicht, dass ein Fluid, beispielsweise ein Reaktionsprodukt oder eine Kohlenwasserstofffraktion, von dem einen zu dem an-deren der beiden Bereiche strömen kann, unbeachtlich etwaiger zwischengeschalteter Bereiche, Bauteile oder benötigter Fördermittel.

Unter einem Mittel wird eine Sache verstanden, die die Erreichung eines Zieles ermöglicht oder dabei behilflich ist. Insbesondere werden unter Mitteln zum Durchführen eines bestimmten Verfahrensschrittes alle diejenigen physischen Gegenstände verstanden, die der Fachmann in Betracht ziehen würde, um diesen Verfahrensschritt durchführen zu können. Beispielsweise wird der Fachmann als Mittel zum Einleiten oder Ausleiten eines Stoffstroms alle Transport- und Fördervorrichtungen, also z. B. Rohrleitungen, Pumpen, Verdichter, Ventile, in Betracht ziehen, die ihm aufgrund seines Fachwissens zur Durchführung dieses Verfahrensschrittes notwendig oder sinnvoll erscheinen.

Unter Oxygenaten werden grundsätzlich alle sauerstoffhaltigen Kohlenwasserstoffverbindungen verstanden, die sich unter Oxygenatumsetzungsbedingungen zu Olefinen, insbesondere zu kurzkettigen Olefinen wie Propylen, und weiteren Kohlenwasserstoffprodukten umsetzen lassen.

Als kurzkettige Olefine werden im Rahmen der vorliegenden Erfindung insbesondere die Olefine verstanden, die bei Umgebungsbedingungen gasförmig vorliegen, beispielsweise Ethylen, Propylen sowie die isomeren Butene 1-Buten, cis-2-Buten, trans-2-Buten, iso-Buten.

Als höhere Kohlenwasserstoffe werden im Rahmen der vorliegenden Erfindung insbesondere diejenigen Kohlenwasserstoffe verstanden, die bei Umgebungsbedingungen flüssig vorliegen.

Zur Bezeichnung von Kohlenwasserstofffraktionen wird folgende Nomenklatur verwendet: "Cn Fraktion" bezeichnet eine Kohlenwasserstofffraktion, die überwiegend Kohlenwasserstoffe der C-Kettenlänge n, also mit n C-Atomen, enthält. "Cn-Fraktion" bezeichnet eine Kohlenwasserstofffraktion, die überwiegend Kohlenwasserstoffe der C-Kettenlänge n sowie mit niedrigeren C-Kettenlängen enthält. "Cn+ Fraktion" bezeichnet eine Kohlenwasserstofffraktion, die überwiegend Kohlenwasserstoffe der C-Kettenlänge n sowie mit höheren C-Kettenlängen enthält. Aufgrund der verwendeten physikalischen Trennverfahren, beispielsweise der Destillation, ist die Auftrennung hinsichtlich der C-Kettenlänge nicht in der Weise zu verstehen, dass Kohlenwasserstoffe mit anderer Kettenlänge rigoros ausgeschlossen werden. So enthält eine Cn- Fraktion je nach Verfahrensbedingungen des Trennverfahrens immer noch geringe Mengen von Kohlenwasserstoffen mit einer C-Zahl größer als n.

Die genannten Aggregatzustände fest, flüssig und gasförmig bzw. dampfförmig sind immer in Bezug auf die lokalen physikalischen Bedingungen zu verstehen, die bei dem jeweiligen Verfahrensschritt oder in dem jeweiligen Anlagenteil herrschen, sofern nichts anderes angegeben ist. Im Rahmen der vorliegende Anmeldung sind die Aggregatzustände gasförmig bzw. dampfförmig als synonym zu betrachten.

Unter einem Auftrennen eines Stoffstroms ist im Zusammenhang mit der vorliegenden Erfindung eine Aufteilung des Stroms in mindestens zwei Teilströme zu verstehen. Wenn nichts anderes angegeben wird, ist davon auszugehen, dass die stoffliche Zusammensetzung der Teilströme derjenigen des Ausgangsstroms entspricht, außer für die Fälle, bei denen dem Fachmann unmittelbar einsichtig ist, dass eine Änderung der stofflichen Zusammensetzung der Teilströme infolge der Bedingungen der Auftrennung zwangsweise auftreten muss.

Unter einer Benzinfraktion ist ein überwiegend, bevorzugt weitgehend vollständig aus höheren Kohlenwasserstoffen bestehendes, unter Umgebungsbedingungen flüssig vorliegendes Stoffgemisch zu verstehen, das geeignet sein kann, als Ottokraftstoff verwendet zu werden.

Unter dem überwiegenden Teil einer Fraktion, eines Stoffstroms etc. ist ein Anteil zu verstehen, der mengenmäßig größer ist als alle anderen jeweils für sich betrachteten Anteile. Insbesondere bei binären Gemischen oder bei der Auftrennung einer Fraktion in zwei Teile ist darunter ein Anteil von mehr als 50 Gew.-% zu verstehen, sofern im konkreten Fall nichts anderes angegeben ist.

Unter der Angabe, dass ein Stoffstrom überwiegend aus einer Komponente oder Komponentengruppe besteht, ist zu verstehen, dass der Stoffmengenanteil (Molenbruch) oder Massenanteil (Massenbruch) dieser Komponente oder Komponentengruppe mengenmäßig größer ist als alle anderen jeweils für sich betrachteten Anteile anderer Komponenten oder Komponentengruppen in dem Stoffstrom. Insbesondere bei binären Gemischen ist darunter ein Anteil von mehr als 50 % zu verstehen. Sofern im konkreten Fall nichts anderes angegeben ist, wird hierbei der Massenanteil (Massenbruch) zugrunde gelegt.

Erfindungsgemäß erfolgt in einem ersten Verfahrensschritt (i) eine heterogen-katalysierte Umsetzung der Oxygenate zu einem Produktstrom, der Wasser, Kohlenwasserstoffe und wenigstens ein Oxygenat enthält. Anschließend wird in einem zweiten Schritt (ii) dieser Produktstrom in wenigstens einer Quenchstufe gequencht, wodurch ein gasförmige Kohlenwasserstoffe und Olefine umfassender Strom G, ein flüssiger, zum überwiegenden Teil aus Wasser und Oxygenaten bestehender wässriger Strom W und ein flüssiger, zu überwiegenden Teil aus Kohlenwasserstoffen bestehender Strom O erhalten wird. Strom G und Strom O werden sodann gemeinsam oder separat in einem dritten Schritt (iii) aufgetrennt in einen Strom C4-, welcher zum überwiegenden Teil aus Kohlenwasserstoffen mit vier oder weniger Kohlenstoffatomen besteht, und einen Strom C4+, welcher zum überwiegenden Teil aus Kohlenwasserstoffen mit vier oder mehr Kohlenstoffatomen besteht. Möglich ist es auch, dass nur Strom G oder Strom O dieser Auftrennung unterzogen werden.

Entscheidend ist es, dass der im dritten Schritt (iii) erhaltene Strom C4+ schließlich als Recyclestrom direkt zu Schritt (i) zurückgeführt und/oder in eine Benzinfraktion geleitet wird.

Verfahrensschritt (iii) kann für jede Art von Oxygenat-zu Olefin-Anlage (OTO-Anlage) angewendet werden und ist unabhängig von der Auslegung und Konzeption der vorgeschalteten Reaktionssektion, der Reaktionswärmerückgewinnungssektion, der Kühl- und Wassertrennsektion (Quenchsystem) und der Verdichtungssektion. Entscheidend ist vielmehr, dass durch die direkte Rückführung des C4+ Stroms, gewonnen als Debutanizer-Sumpfprodukt, insbesondere des überwiegenden Teils, also bevorzugt mehr als 50 Gew.-%, besonders bevorzugt mehr als 75 Gew.-% des Debutanizer-Sumpfprodukts, und/oder durch die direkte Abzweigung in die Benzinfraktion die Abtrennung von schweren Kohlenwasserstoffen signifikant vereinfacht werden kann. Im Vergleich dazu wird im konventionellen Verfahren dieser Strom zu einer weiteren Kolonne (Dehexanizer) zur Erzeugung eines C5/C6 Recyclestrom im Kopf der Kolonne und eines C5+ Benzinproduktes aus dem Sumpf verarbeitet. Ein Teil des Kopfproduktes wird in einer Benzin-Stabilisatorkolonne weiterverarbeitet, die einen Sumpfproduktstrom erzeugt, der zur Einstellung des Siedebereichs des Benzins verwendet wird.

In diesem Zusammenhang haben Untersuchungen gezeigt, dass das Recycle von schweren Kohlenwasserstoffen, die C7+ Kohlenwasserstoffe enthalten, keinen negativen Einfluss auf die Propylenausbeute hat, solange eine kleine Menge von schweren Kohlenwasserstoffen aus dem zirkulierenden Kohlenwasserstoffstrom als Spülstrom oder Purgestrom entfernt wird, was bevorzugt kontinuierlich oder periodisch erfolgt. Die Rückführung der C7+ Kohlenwasserstoffe in den Reaktor führt vielmehr noch zu einer Steigerung der Propylenausbeute.

Bevorzugt wird in dem erfinderischen Verfahren der größte Teil des C4+ Stroms direkt verdampft und in den OTO-Reaktor zurückgeführt. Nur ein bei der Verdampfung verbliebener, flüssiger Anteil und ein kleinerer Teil des Debutanizer-Sumpfstromstroms, in Summe bevorzugt weniger als 25 Gew.-%, besonders bevorzugt weniger als 10 Gew.-% des Gesamtstroms als Summe aus dem Recyclestroms und dem Benzinfraktions-Kohlenwasserstoffstrom wird einer Benzin-Stabilisatorkolonne zugeführt. Somit entfällt eine Kolonne zur Abtrennung der C6+ Fraktion mit den entsprechenden Wärmetauschern und Pumpen. Die Verdampfung des Recyclestroms kann günstiger Weise als Teilverdampfung mit einer Abschlämmung ausgestaltet werden, die auf die Benzin-Stabilisatorkolonne übertragen wird oder einen Bestandteil letzterer bildet. Optional kann ein Stripper eingesetzt werden, der den verdampften Anteil von dem flüssigen Anteil abtrennt, um die Anreicherung unerwünschter schwerer Kohlenwasserstoffe zu verhindern.

Ein weiterer Vorteil des erfindungsgemäßen Verfahrens besteht darin, dass die unerwünschten schweren Kohlenwasserstoffe, die bei dem Quenchschritt durch Kondensation erhalten werden, nicht durch weitere Kolonnen geführt, sondern direkt zur Benzinfraktion geführt werden. Dies reduziert die Belastung vorhandener Kolonnen (vor allem des Debutanizers) und damit den Energieaufwand, die benötigte Kolonnengröße und die Verschmutzungsneigung auf den Böden. Gegebenenfalls werden sogar Kolonnen eingespart.

Ein Aspekt der Erfindung besteht also darin, die Reinigungssektion gemäß Stand der Technik zu vereinfachen, ohne Einbußen an Propylen zu erleiden, die durch die Erzeugung eines schwereren Recyclestroms gemäß Stand der Technik hervorgerufen werden. Überraschenderweise ist die mit dem erfindungsgemäßen Verfahren erhaltene Zusammensetzung der Recycleströme hilfreich für den Prozess und erlaubt es, die Olefinproduktion des OTO-Reaktors zu erhöhen. Durch das direkte Recycle des größten Teils des Debutanizer-Sumpfprodukts und/oder seiner direkten Zuführung in die Benzinfraktion werden daher Einsparungen bei den Investitionskosten und Betriebskosten ohne Beeinträchtigung der Propylenausbeute erzielt.

Da das Kohlenwasserstoffrecycle eine wichtige Rolle für die Propylenausbeute der OTO-Anlage spielt, werden sowohl die Gesamtdurchflussmenge als auch die Zusammensetzung sorgfältig gesteuert oder geregelt. Beispielsweise kann das MTP-Verfahren für jedes Verhältnis von Kohlenwasserstoffrecycle zu Reaktoreinspeisung Methanol von 0,75 bis 1,5 ausgelegt werden. Die Aufteilung der verschiedenen Ströme kann über die Lebensdauer des Katalysators angepasst werden, um die für die Propylenausbeute vorteilhaftesten Ströme zu maximieren.

### Weitere bevorzugte Ausgestaltungen der Erfindung

Es hat sich weiterhin als günstig herausgestellt, wenn während des Quenchens (Schritt (ii)) ein weiterer flüssiger, zum überwiegenden Teil aus Kohlenwasserstoffen mit vier oder mehr Kohlenstoffatomen bestehender Strom S erhalten wird, so dass bereits hierbei sehr schwere Kohlenwasserstofffraktionen abgetrennt werden können.

Besonders günstig ist es, wenn der Strom S mit dem Strom C4+ zusammengeführt wird und/oder zumindest ein Teil des Stroms S in die Benzinfraktion geleitet wird. Dadurch wird der Strom S ebenfalls Teil des Wertproduktes Benzin.

Für das Quenchsystem bedeutet das, dass das gekühlte OTO-Reaktorabwasser in das Quench-System geleitet wird, wo das Reaktorabwasser weiter gekühlt und der größte Teil des Wassers kondensiert und vom Kohlenwasserstoffprodukt getrennt wird. Die nicht kondensierten Kohlenwasserstoffe verlassen die vorzugsweise vorgesehenen Pre-Quench- und Quench-Kolonnen als Überkopfdampf. Abhängig von der Konfiguration des Quenchsystems und dem Katalysatorbetrieb kann ein kleiner Strom von flüssigen Kohlenwasserstoffen im Quenchsystem erzeugt werden. Diese werden an die nachgeschaltete Reinigungssektion weitergeleitet. Der größte Teil des Quenchwassers wird im Quenchwasserkreislauf zirkuliert.

Das entstehende Wasser kann zur Wärmeintegration an verschiedenen Stellen des Prozesses genutzt werden. Nach der Endabkühlung wird das Wasser dann in den Quench zurückgeführt. Ein Teil des Quenchwassers verlässt bevorzugt diesen Kreislauf und wird zur Reinigung und Rückgewinnung von nicht umgesetzten Oxygenaten in die Methanol-Rückgewinnungskolonne geleitet. Ein Teil des in der Methanol-Rückgewinnungskolonne gereinigten Wassers kann auch als Feed für die Prozessdampferzeugung verwendet werden, wodurch ein Prozessdampfrecycle entsteht. Der Prozessdampf wird günstigerweise so erzeugt, dass keine Katalysatorgifte, z. B. Natrium, in dem dem MTP-Reaktor zugeführten Strom verbleiben.

Der Überschuss an gereinigtem Wasser (Reaktionswasser) verlässt das System für den nachgeschalteten internen Gebrauch oder die Weiterverarbeitung und den externen Gebrauch. Mögliche externe Nutzungen des Reaktionswassers umfassen: Kühlwassernachspeisung, Rückführung zur Synthesegaserzeugung, weitere Reinigung für den Einsatz als Kesselspeisewasser-Ergänzungswasser oder Bewässerungswasser.

Ein Nebenprodukt der MTP-Reaktion sind organische Säuren. Bei Bedarf kann dem Quenchwasser eine pH-regulierende Chemikalie zugegeben werden, um den pH-Wert zu kontrollieren und so die Korrosionsneigung zu minimieren. Die Methanol-Rückgewinnungskolonne erhält zusätzliche Ströme aus der Reinigung, die hauptsächlich Oxygenate und Wasser enthalten. Dazu gehören unter anderem Ströme aus der Extraktion, die zur Entfernung von Oxygenaten aus verschiedenen Produktströmen eingesetzt werden.

Eine ergänzende oder alternative Ausgestaltung der Erfindung sieht vor, dass dass Strom G und/oder Strom O einem bevorzugt mehrstufigen Kompressionsschritt zugeführt werden und aus diesem Kompressionsschritt, bevorzugt nach der ersten Stufe, ein zum überwiegenden Teil aus Kohlenwasserstoffen mit 4 oder mehr Kohlenstoffatomen bestehender, flüssiger Strom K erhalten wird. Dadurch können die nachgeschalteten Reinigungsstufen von langkettigen Kohlenwasserstoffen entlastet werden, so dass sich in diesen, vorzugsweise als Rektifikationskolonnen ausgestalteten Einheiten weniger Verschmutzungen pro Zeiteinheit ansammeln.

Für diese Abtrennung eines Stroms K ist es besonders vorteilhaft, wenn der Strom K dem Strom S und/oder dem Strom C4+ zugemischt wird und/oder zumindest ein Teil des Stroms K direkt in die Benzinfraktion geleitet wird. Dabei sorgt eine optionale Stabilisierung der Benzinfraktion mittels einer entsprechenden Stabilisatorkolonne dafür, dass das Benzinprodukt in einem atmosphärischen Tank gelagert werden kann, wodurch sowohl der Strom S als auch der Strom K ausbeutesteigernd eingesetzt werden können.

Vorteilhaft ist es zudem, wenn in dem Kompressionsschritt eine weitere flüssige Phase L erhalten wird, welche in Schritt (iii) eingeleitet wird. Diese flüssige Phase weist, besonders bei einer Abtrennung eines Stroms S im Quench und K in der Kompression, schon eine verhältnismäßig hohe Reinheit bezogen auf langkettige Kohlenwasserstoffe mit wenigstens fünf C-Atomen auf.

Besonders günstig ist eine Ausgestaltung der Kompression, in der das Kohlenwasserstoffdampfprodukt aus dem Quench-System in mehreren, bevorzugt in mindestens drei, meist bevorzugt in vier Stufen verdichtet wird. Nach jeder Verdichtungsstufe wird das komprimierte Gas abgekühlt und teilweise kondensiert. Die verdichteten dampfförmigen und flüssigen Kohlenwasserstoffe werden der Reinigungsanlage zugeführt. Das flüssige Wasser wird zu dem Quench-System zurückgeführt. Die Trennung von Wasser und flüssigen Kohlenwasserstoffen kann entweder einzeln nach jeder Kompressionsstufe erfolgen, wobei zwischen den Verdichterstufen Kondenswasserströme erzeugt werden und/oder in einem gemeinsamen Schritt, nachdem die Ströme aus allen Stufen gemischt wurden.

Um Ablagerungen auf dem Kompressor zu vermeiden, kann es zudem vorteilhaft sein, den Kompressor kontinuierlich oder intermittierend zu waschen. Hierzu kann ein interner Strom mit geeigneter Zusammensetzung, z. B. die erhaltenen Benzinfraktion, verwendet werden. Zusätzliche Maßnahmen können ergriffen werden, um Sauerstoffverbindungen aus den Kohlenwasserstoffströmen zu entfernen. Diese umfassen das Waschen des Dampfes mit Wasser und/oder die Extraktion in der Flüssigkeit mit Wasser. Das sauerstoffreiche Wasser wird zur Trennung in die Methanolrückgewinnung zurückgeführt.

Eine weitere Ausgestaltung der Erfindung sieht vor, dass der Strom C4+ mindestens teilweise verdampft und der entstehende dampfförmige Strom D in Schritt (i) zurückgeführt wird. Dadurch muss in der Abtrennung der C4- Fraktion von dem C4+ Strom weniger Energie zugeführt werden. Dadurch wird die anschließende Auftrennung (Debutanizer) weiter entlastet.

Ist nach der Kompression eine partielle Verdampfung vorgesehen, so verbleibt auch ein flüssiger Strom F, der bevorzugt zumindest teilweise dem Strom S und/oder dem Strom K und/oder dem Strom C4+ zugemischt und/oder zumindest ein Teil direkt in die Benzinfraktion geleitet wird. So werden auch hier die verhältnismäßig langkettigen Kohlenwasserstoffe werterhaltend genutzt.

Je nach Kühlleistung nach dem Kompressor und den Produktanforderungen für das C4 haltige Produkt kann der Dampfstrom aus der Kompression auch direkt dem Schritt (iii) zur weiteren Auftrennung der C4 Fraktion zugeführt werden.

Bevorzugt folgt auf Schritt (iii) wenigstens ein weiterer Aufreinigungsschritt, in dem der Strom C4- in einen zum überwiegenden Teil aus Kohlenwasserstoffen mit drei Kohlenstoffatomen bestehenden und Propylen umfassenden Strom C3 und in einen zum überwiegenden Teil aus Kohlenwasserstoffen mit höchstens zwei Kohlenstoffatomen bestehenden und Ethen umfassenden Strom C2- aufgetrennt wird.

Dabei kann das dampfförmige Kopfprodukt und optional der Kohlenwasserstoffdampf aus dem Kompressor einer nachgeschalteten Reinigungsstufe (Depropanizer) zugeführt werden. Dort werden C3- Kohlenwasserstoffe von C4+ Kohlenwasserstoffen getrennt. Zusätzlich werden Restmengen an DME und anderen Oxygenaten aus den Kohlenwasserstoffen durch Zugabe von reinem Methanol (z.B. Grade-AA-Methanol) als Waschmittel entfernt. Detaillierte Optionen für die Extraktivdestillation sind in der DE 10 2004 052 658 offenbart.

Das Sumpfprodukt des Depropanizers, der Strom C4, wird gekühlt und in eine C4-Extraktionssektion geleitet. Hier werden Absorptionsmethanol und absorbierter DME aus dem gekühlten Strom C4 extrahiert. Dies erfolgt vorzugsweise mehrschrittig, wobei zuerst eine Mixer-Settler-(Mischabsetzer)-Kombination vorgesehen ist, in der das Methanol und ein Teil des DME abgetrennt wird. Das Wasser-Oxygenat-Gemisch wird dann der Methanol-Rückgewinnungssektion zur Auftrennung zugeführt.

Der C4 Strom wird verdampft und als Rücklauf in den OTO-Reaktor zurückgeführt. Bei Bedarf kann eine weitere Entfernung von Oxygenaten aus dem C4 Strom oder einem Teilstrom daraus mit einem C4 Extraktor vorgesehen werden. Das Wasser-Oxygenat-Gemisch wird ebenfalls der Methanol-Rückgewinnungssektion zur Trennung zugeführt. Das Kopfprodukt des Extraktors, das die C4 Kohlenwasserstofffraktion enthält, die von Methanol und DME befreit wurde, wird mit dem Propan aus anderen Trennschritten kombiniert und stellt ein Wertprodukt (LPG-Produkt) dar. Alternativ können weitere C4 und C3 Produktströme gewonnen werden. Eine Alternative zur Oxygenatentfernung wird ebenfalls in der DE 10 2004 052 658 gelehrt.

Das Kopfprodukt des Depropanizers, der C3- Strom, ist frei von oder sehr arm an DME oder anderen Oxygenatkomponenten. Die Dampfphase des Depropanizers wird zu optional getrocknet und sodann einer nachgeschalteten Kolonne (Deethanizer) zugeführt.

Üblicherweise dem Depropanizer nachgeschaltet, jedenfalls aber nach Schritt (iii), folgt ein weiterer Aufreinigungsschritt, in dem ein überwiegend, bevorzugt zu mindestens 70 Gew.-%, meist bevorzugt zu wenigstens 90 Gew.-% aus Kohlenwasserstoffen mit drei Kohlenstoffatomen bestehender Strom C3 und ein überwiegend, bevorzugt zu mindestens 70 Gew.-%, meist bevorzugt zu wenigstens 90 Gew.-% aus Kohlenwasserstoffen mit höchstens zwei Kohlenstoffatomen bestehender Strom C2- voneinander getrennt werden. Bevorzugt wird hier die C3 Kohlenwasserstofffraktion aus dem Depropanizer gekühlt und teilweise kondensiert, bevor sie dieser Trennstufe (Deethanizer) zugeführt wird. In dieser Kolonne wird die C3 Kohlenwasserstofffraktion in einen C2- Strom, der über Kopf gewonnen wird, und einen C3 Strom, der am Sumpf entnommen wird, aufgeteilt.

Der Druck im Rücklaufsystem des Deethanizers muss auf das verfügbare Kühlmedium im Verflüssiger eingestellt und im vorgeschalteten Verdichter verdichtet werden. Eine Möglichkeit besteht darin, den Debutanizer, Depropanizer und Deethanizer mit ca. 20 bara (bar, absolut) zu betreiben und im Deethanizer-Kondensator ein Versorgungsmedium zu verwenden, das wesentlich kälter ist als Propylen-Kühlmittel (< - 40°C). Eine zweite Möglichkeit besteht darin, den Depropanizer und Deethanizer oder den Deethanizer nur mit einem Druck zu betreiben, der den Einsatz von Propylen-Kältemittel im Deethanizer-Kondensator erlaubt. Eine dritte Möglichkeit besteht darin, alle Säulen mit ca. 20 bara zu betreiben und nur einen Kompressor für den Kopfstrom des Deethanizers zu verwenden, um Propylen-Kältemittel als Betriebsmittel im Deethanizer-Kondensator zu verwenden.

Eine kleine Menge Kohlendioxid (CO2) ist als ungewünschte Nebenkomponente im Kopfprodukt des Deethanizers enthalten. Um diese CO2-Komponenten zu eliminieren, wird der am Kopf der Kolonne abgezogene Strom in einen CO2-Wäscher geleitet. Zur Entfernung von CO2 aus dem C2 Strom können zwei oder mehr Laugekreisläufe verwendet werden. Normalerweise verhindert eine abschließende Waschzone mit demineralisiertem Wasser den Durchbruch von Lauge. Das aus dem CO2-Wäscher austretende Gas kann dann als C2 Recycle genutzt werden.

Das Sumpfprodukt aus dem Deethanizer wird einem C3 Splitter zugeführt, um das Propylenprodukt vom Nebenprodukt Propan in der gewünschten Reinheit zu trennen. Abhängig von den genauen Spezifikationen zur Verwendung des monomeren Propylens, z. B. zur Herstellung von Polypropylen, schließen sich zusätzliche Reinigungsschritte an.

An verschiedenen Positionen kann zudem Ethylen entnommen werden, z. B. als nicht kondensierbare Flüssigkeit aus dem Deethanizer-Kondensator. Wenn CO₂ aus diesem Strom entfernt werden muss, können durch partielle Kondensation des Recycles und Spülen der nicht kondensierbaren Stoffe die leichten Komponenten angesammelt werden. Die Reinigung des Ethylens erfolgt durch eine Entfernung von Wasser und CO₂ zur Vermeidung von Eis- und Hydratbildung. Anschließend wird der Strom einem Demethanizer zugeführt. Das Kopfprodukt des Demethanizers kann als Brenngas verwendet werden, während das Sumpfprodukt vom Demethanizer in einen C₂ Splitter weitergeleitet wird, in dem Ethylen in einer für die Polymerherstellung geeigneten Qualität abgetrennt wird. Das hier abgetrennte Ethan kann ebenfalls als Brenngas verwendet werden.

Um eine Polymerisation zu verhindern, kann ein Inhibitor zu den Kondensatoren und Reboilern wenigstens einer Trennkolonne, insbesondere dem Debutanizer, Dehexanizer und einer ggf. vorhandenen Benzin-Stabilisatorkolonne hinzugefügt werden.

Die Erfindung umfasst weiterhin auch die Verwendung einer Anlage zur Durchführung des erfindungsgemäßen Verfahrens. Eine solche Verwendung weist die Merkmale des Anspruchs 10 auf.

Im Detail umfasst eine solche Anlage zur Herstellung von Olefinen aus Oxygenaten einen Reaktor zur heterogen-katalysierten Umsetzung der Oxygenate zu einem Produktstrom, der Wasser, Kohlenwasserstoffe und wenigstens ein Oxygenat enthält, wenigstens eine Quenchstufe zum Quenchen des Produktstroms, wodurch ein gasförmige Kohlenwasserstoffe und Olefine umfassender Strom G, ein flüssiger, zum überwiegenden Teil aus Wasser und Oxygenaten bestehender wässriger Strom W und ein flüssiger, zum überwiegenden Teil aus Kohlenwasserstoffen bestehender und Olefine umfassender Strom O erhalten wird, ferner umfassend eine Trennvorrichtung zum Auftrennen des zum überwiegenden Teil aus Kohlenwasserstoffen bestehenden Stroms O in einen Strom C4-, welcher zum überwiegenden Teil aus Kohlenwasserstoffen mit vier oder weniger Kohlenstoffatomen besteht, und einen Strom C4+, welcher zum überwiegenden Teil aus Kohlenwasserstoffen mit vier oder mehr Kohlenstoffatomen besteht. Gekennzeichnet ist die erfindungsgemäße Anlage durch wenigstens eine Rückführleitung, mit der mindestens einen Teil des Stroms C4+ zum Reaktor zurückgeführt wird und/oder eine Leitung, durch die zumindest ein Teil des Stroms C4+ in eine Benzinfraktion geleitet wird.

Ein solche Anlage weist deutlich geringere Investitions- und Betriebskosten auf, da im Vergleich zu entsprechenden Anlagen gemäß Stand der Technik mindestens eine Trennvorrichtung entfallen kann.

Weitere, besondere Ausgestaltungen sind den entsprechenden abhängigen Ansprüchen zu entnehmen. Ihre Eigenschaften und Vorteile wurden bereits bei der Beschreibung entsprechender Verfahrensausgestaltungen genannt.

### Ausführungsbeispiele

Weitere Merkmale, Vorteile und Anwendungsmöglichkeiten der Erfindung ergeben sich aus der nachfolgenden Beschreibung eines Ausführungsbeispiels und der Zeichnungen.

Es zeigen:
- Fig. 1: eine schematische Darstellung eines Verfahrens bzw. einer Anlage gemäß Stand der Technik,
- Fig. 2: eine schematische Darstellung eines Verfahrens bzw. einer Anlage gemäß der Erfindung.

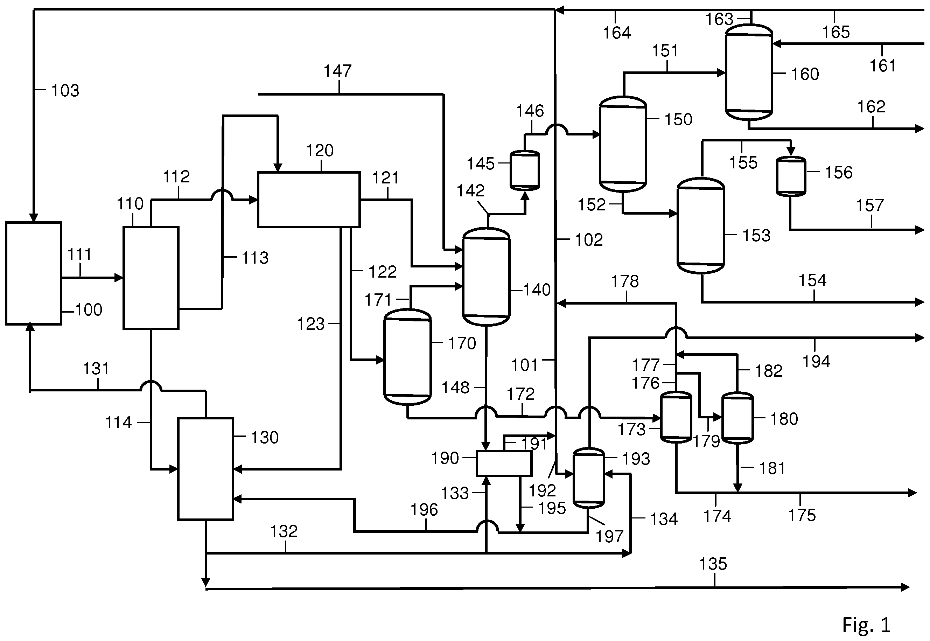

Figur 1 zeigt als Sonderfall einer OTO-Anlage den Aufbau einer MTP-Anlage, wie sie aus dem Stand der Technik bekannt ist. Der OTO-Reaktor 100, in dem die Bildung von Olefinen aus Oxygenaten wie beispielsweise Methanol und/oder Dimethylether (DME) abläuft, wird über eine nicht gezeigte Frischfeed-Leitung mit Oxygenaten sowie Wasserdampf als Verdünnungsmittel beaufschlagt; ferner werden über Leitungen 103 und 131 auch Recycleströme zum Reaktor zurückgeführt. Über Leitung 111 wird der Produktstrom des Reaktors 100 in das Quench-system 110 eingebracht. Eine im wesentlichen Wasser enthaltene Phase wird über Leitung 114 aus dem Quenchsystem 110 abgeführt und in eine Methanolrückgewinngungseinheit 130 eingebracht. Die im Quenchsystem 110 abgetrennte gasförmige Phase gelangt über Leitung 112 in einen Kompressor 120. Über Leitung 113 wird zudem eine flüssige, im wesentlichen aus Kohlenwasserstoffen bestehende Phase aus dem Quenchsystem 110 in den Kompressor 120 eingespeist.

Aus dem Kompressor 120 wird ein flüssiger Strom über eine Leitung 121 in eine Trennvorrichtung 140 geführt. In dieser Trennvorrichtung 140 wird die C3- Fraktion von der C4 Fraktion getrennt, weshalb sie auch als Depropanizer bezeichnet wird. Die Trennvorrichtung 140 ist vorzugsweise als Trennkolonne ausgestaltet. Vorzugsweise erfolgt die Rektifikation in der Trennvorrichtung 140 als Extraktivdestillation, weshalb über Leitung 147 ein Extraktionsmittel, beispielsweise Methanol, zugeführt werden kann, soweit dies gewünscht ist.

Das über Kopf abgezogene Kopfprodukt, welches die C3- Fraktion enthält, wird über Leitung 142 einem Trockner 145 zugeführt, von dem aus es weiter über Leitung 146 in eine weitere Trennvorrichtung 150 gelangt. Auch eine direkte Verbindung von Trennvorrichtung 140 mit Trennvorrichtung 150 ist möglich. In dieser Trennvorrichtung 150 wird die C3 Fraktion von der C2- Fraktion getrennt, weshalb die Trennvorrichtung 150 auch als Deethanizer bezeichnet wird.

Über Leitung 151 gelangt das Kopfprodukt der Trennvorrichtung 150 in eine Trennvorrichtung 160, welche vorzugsweise als Wäscher ausgebildet ist. Bei einer Ausbildung als Wäscher wird über Leitung 161 ein Waschmittel, beispielsweise Waschwasser eingebracht und über Leitung 162 wieder abgezogen. Der enthaltene C2- Strom wird mit Leitung 163 über Kopf abgezogen und kann über Leitung 164 einem zurück in den Reaktor 100 führenden Recyclestrom in Leitung 103 zugeführt werden und/oder über Leitung 165 aus dem System ausgeleitet und z. B. einer Brenngasnutzung oder einer weiteren Trennvorrichtung zur Aufreinigung der enthaltenen Produkte zugeführt werden, um beispielsweise enthaltenes Ethylen als Reinprodukt zu gewinnen.

Das Sumpfprodukt der Trennvorrichtung 150 wird über Leitung 152 einer Trennvorrichtung 153 zugeführt, welche Propan von Propylen trennt. In diesem sogenannten C3 Splitter wird über Leitung 154 Propan aus dem System abgezogen, welches z. B. als Brenngas Verwendung finden kann. Über Leitung 155 wird das Zielprodukt Propylen abgezogen, welches optional in Abhängigkeit von Qualitätsanforderungen einer weiteren Aufreinigungsvorrichtung 156 zugeführt werden kann, aus der es dann über Leitung 157 ausgeleitet wird. Die Aufreinigungsvorrichtung 156 kann beispielsweise mit einem für Oxygenate selektiven Sorbens befüllt werden, so dass Oxygenate aus dem Propylen-Produktstrom je nach Spezifikation bis auf geringe Spuren entfernt werden können.

Über Leitung 122 wird aus dem Kompressor eine flüssige Phase abgezogen, welche einer Trennvorrichtung 170 zugeführt wird, in der die C4- Fraktion und die Oxygenate von der C4+ Fraktion getrennt werden. Diese Trennvorrichtung 170 wird auch als Debutanizer bezeichnet. Über Leitung 171 gelangt die C4- Fraktion und das Oxygenat enthaltende Kopfprodukt der Trennvorrichtung 170 in die Trennvorrichtung 140.

Über Leitung 172 wird das Sumpfprodukt der Trennvorrichtung 170 in eine weitere Trennvorrichtung 173 gegeben, aus der die C7+ Fraktion von der C6 Fraktion getrennt wird, weshalb die Trennvorrichtung 173 auch als Dehexanizer bezeichnet wird. Das Sumpfprodukt wird über Leitung 174, 175 abgezogen. Über Leitung 176, 177 und 178 kann ein Recyclestrom einer Leitung 101 zugeführt werden, die über Leitungen 102 und 103 zurück zum Reaktor 100 führt.

Aus Leitung 176 kann überdies eine Leitung 179 abgezweigt werden, welche in eine Trennvorrichtung 180 (Benzin-Stabilisatorkolonne) geführt werden. Das hier erhaltene Benzin wird als Wertprodukt über Leitung 181 und Leitung 175 ausgeschleust. Die über Kopf abgetrennten C4 Kohlenwasserstoffe werden über Leitung 182 den Leitungen 177 und 178 zugeführt, so dass sie letztendlich in den Recyclestrom in Leitungen 102, 103 münden.

Das Sumpfprodukt der Trennvorrichtung 140 wird über Leitung 148 abgezogen und gelangt so in eine Mixer-Settler-Kombination 190. Aus dieser werden Kohlenwasserstoffe über Leitung 191 abgezogen und können vollständig oder teilweise über Leitungen 101, 102, 103 in den Reaktor 100 zurückgeführt werden. Alternativ oder ergänzend ist es möglich, die Kohlenwasserstoffe über Leitung 192 einer Trennvorrichtung 193 zuzuführen, welche vorzugsweise als Extraktion ausgestaltet ist. Das Kopfprodukt aus der Trennvorrichtung 193 wird über Leitung 194 als Brenngas ausgeschleust.

Aus der Methanol-Dimethylether-Rückgewinnung 130 wird zum einen ein Oxygenat-Recyclestrom über Leitung 131 in den Reaktor 100 zurückgeführt. Über Leitungen 132 und 133 kann optional mit Methanol und/oder mit Dimethylether beladenes Wasser in die Mixer-Settler-Einheit 190 gespeist werden, aus der es dann auch über die Leitung 195 wieder der Leitung 197 und 196 zugeführt werden kann.

Über Leitungen 132, 134 kann das Wasser als Extraktionsmittel in die Trennvorrichtung 193 geführt werden, sofern diese als Extraktion ausgestaltet ist, aus der es dann auch über die Leitung 197 wieder der Leitung 196 zugeführt werden kann. Über Leitung 135 kann schließlich das Wasser aus dem System ausgeleitet werden.

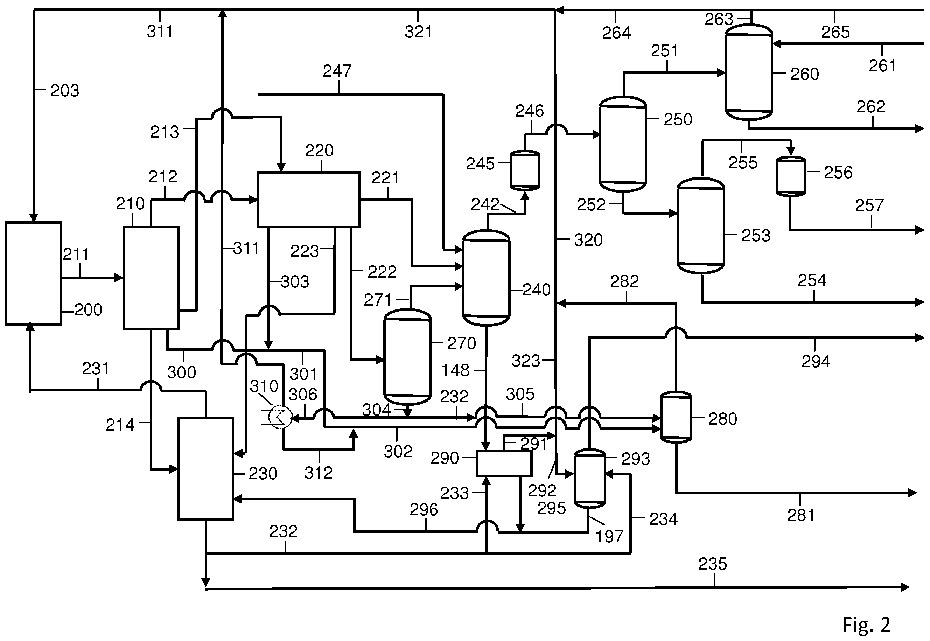

Fig. 2 zeigt eine erfindungsgemäß ausgestaltete Anlage zur Durchführung des erfindungsgemäßen Verfahrens. Dabei entspricht der Komplex aus Reaktor 200 und Quenchsystem 210 im Wesentlichen der in Zusammenhang mit Fig. 1 erläuterten Beschreibung.

Erfindungsgemäß wird in Fig. 2 über Leitungen 300, 301 und 302 direkt ein flüssiger, kohlenwasserstoffhaltiger zweiter Strom der Benzin-Stabilisatorkolonne 280 zugeführt. Der gasförmige Quenchstrom 212 wird weiterhin ebenso der Kompression 220 zugeführt, wie ein erster flüssiger Strom 213, was dem Vorgehen in der Anlage gemäß Stand der Technik, Fig. 1, entspricht.

Abweichend zu dem aus dem in Fig. 1 dargestellten Vorgehen kann optional aus der Kompression 220 ein weiterer Strom 303 abgezweigt werden, welcher hier in die Leitungen 300, 301 und 302 geführt wird und so ebenfalls in die Benzin-Stabilisatorkolonne 280 gelangt. Gleichermaßen ist es möglich, diesen Strom auch mittels einer separaten Leitung (nicht gezeigt) in die Benzin-Stabilisatorkolonne 280 zu führen.

Weiterhin wird über Leitung 304 aus der Trennvorrichtung 270 das Sumpfprodukt abgezogen, welches entweder vollständig oder teilweise über Leitung 305 in die Benzin-Stabilisatorkolonne 280 geführt wird, und/oder wenigstens teilweise über Leitung 306 in eine optionale zusätzliche Verdampfung 310 gelangt. In dieser speziellen Ausgestaltung mit zusätzlicher Verdampfung werden dampf- bzw. gasförmige Anteile erzeugt und über Leitung 311 mit den Recylingleitungen 321, 322 zusammengeführt. Der flüssige Anteil aus der Verdampfung 310 wird über Leitung 312 in die Leitung 301, 302 eingebracht und gelangt so ebenfalls in die Benzin-Stabilisatorkolonne 280. Ein separates Mischen mit anderen Strömen, z. B. mit dem in Leitung 305 geführten oder ein separates Einbringen in die Benzin-Stabilisatorkolonne 280 ist ebenfalls möglich.

Durch die erfindungsgemäße Verfahrensführung entfällt die Trennvorrichtung 173 mit den zugehörigen Leitung 174 bis 178 vollständig. Die Rückführleitungen 320, 321 und 322 führen somit Ströme mit einer etwas anderen Zusammensetzung.

### Bezugszeichenliste

- 100: OTO-Reaktor
- 101-103: Leitung
- 110: Quench-System
- 111-114: Leitung
- 120: Kompressor
- 121-123: Leitung
- 130: Methanol-DME-Rückgewinnung
- 131-135: Leitung
- 140: Trennvorrichtung
- 142: Leitung
- 150: Trennvorrichtung
- 151, 152: Leitung
- 153: Trennvorrichtung
- 154, 155: Leitung
- 156: Aufreinigungsstufe
- 157: Leitung
- 160: Trennvorrichtung
- 161-165: Leitung
- 170: Trennvorrichtung
- 171, 172: Leitung
- 173: Trennvorrichtung
- 174-179: Leitung
- 180: Trennvorrichtung (Benzin-Stabilisatorkolonne)
- 181, 182: Leitung
- 190: Mixer-Settler-Kombination
- 191,192: Leitung
- 193: Trennvorrichtung
- 194-197: Leitung
- 200: Reaktor
- 210: Quench-System
- 211-214: Leitung
- 220: Kompressor
- 221-223: Leitung
- 230: Methanol-DME-Rückgewinnung
- 231-235: Leitung
- 240: Trennvorrichtung
- 242: Leitung
- 250: Trennvorrichtung
- 251, 252: Leitung
- 253: Trennvorrichtung
- 254, 255: Leitung
- 256: Aufreinigungsstufe
- 257: Leitung
- 260: Trennvorrichtung
- 261-265: Leitung
- 270: Trennvorrichtung
- 271: Leitung
- 280: Trennvorrichtung (Benzin-Stabilisatorkolonne)
- 281, 282: Leitung
- 290: Mixer-Settler-Kombination
- 291, 292: Leitung
- 293: Trennvorrichtung
- 294-297: Leitung
- 300-306: Leitung
- 310: Verdampfer
- 311, 312: Leitung
- 320-323: Leitung

## Patentansprüche

1. Verfahren zur Herstellung von Olefinen aus Methanol und/oder Dimethylether (DME), umfassend die folgenden Schritte:
(i) Heterogen-katalysierte Umsetzung des Methanols und/oder Dimethylethers als Oxygenate in einem Oxygenat-zu-Olefin-Reaktor unter Oxygenatumwandlungsbedingungen zu einem Produktstrom, der Wasser, Kohlenwasserstoffe und wenigstens ein Oxygenat enthält,
(ii) Quenchen des Produktstroms in wenigstens einer Quenchstufe, wodurch ein gasförmige Kohlenwasserstoffe und Olefine umfassender Strom G, ein flüssiger, zum überwiegenden Teil aus Wasser und Oxygenaten bestehender wässriger Strom W und ein flüssiger, zum überwiegenden Teil aus Kohlenwasserstoffen bestehender und Olefine umfassender Strom O erhalten wird,
(iii) Auftrennen des Stroms G und/oder des Stroms O in einen Strom C4-, welcher zum überwiegenden Teil aus Kohlenwasserstoffen mit vier oder weniger Kohlenstoffatomen besteht und Olefine umfasst, und einen Strom C4+, welcher zum überwiegenden Teil aus Kohlenwasserstoffen mit vier oder mehr Kohlenstoffatomen besteht und Olefine umfasst,
**dadurch gekennzeichnet, dass** der in Schritt (iii) erhaltene Strom C4+ direkt über eine Benzin-Stabilisatorkolonne zu Schritt (i) zurückgeführt und/oder in eine Benzinfraktion geleitet wird.

2. Verfahren nach Anspruch 1, **dadurch gekennzeichnet, dass** in Schritt (ii) ein weiterer flüssiger, zum überwiegenden Teil aus Kohlenwasserstoffen mit vier oder mehr Kohlenstoffatomen bestehender Strom S erhalten wird.

3. Verfahren nach Anspruch 2, **dadurch gekennzeichnet, dass** der Strom S mit dem Strom C4+ zusammengeführt wird und/oder zumindest ein Teil des Stroms S in die Benzinfraktion geleitet wird.

4. Verfahren nach einem der vorhergehenden Ansprüche, **dadurch gekennzeichnet, dass** Strom G und/oder Strom O einem bevorzugt mehrstufigen Kompressionsschritt zugeführt werden und aus diesem Kompressionsschritt, bevorzugt nach der ersten Stufe, ein zum überwiegenden Teil aus Kohlenwasserstoffen mit 4 oder mehr Kohlenstoffatomen bestehender, flüssiger Strom K erhalten wird.

5. Verfahren nach Anspruch 4, **dadurch gekennzeichnet, dass** der Strom K dem Strom S und/oder dem Strom C4+ zugemischt wird und/oder zumindest ein Teil des Stroms K direkt in die Benzinfraktion geleitet wird.

6. Verfahren nach Anspruch 4, **dadurch gekennzeichnet, dass** in dem Kompressionsschritt eine weitere flüssige Phase L erhalten wird, welche in Schritt (iii) eingeleitet wird.

7. Verfahren nach einem der vorhergehenden Ansprüche, **dadurch gekennzeichnet, dass** der Strom C4+ mindestens teilweise verdampft und der entstehende dampfförmige Strom D in Schritt (i) zurückgeführt wird.

8. Verfahren nach Anspruch 7, **dadurch gekennzeichnet, dass** durch das teilweise Verdampfen auch ein im flüssigen Zustand verbleibender Strom F erhalten wird, der zumindest teilweise dem Strom S und/oder dem Strom K und/oder dem Strom C4+ zugemischt und/oder zumindest ein Teil direkt in die Benzinfraktion geleitet wird.

9. Verfahren nach einem der vorhergehenden Ansprüche, **dadurch gekennzeichnet, dass** auf Schritt (iii) wenigstens ein weiterer Aufreinigungsschritt folgt, in dem der Strom C4- in einen zum überwiegenden Teil aus Kohlenwasserstoffen mit drei Kohlenstoffatomen bestehenden und Propylen umfassenden Strom C3 und in einen zum überwiegenden Teil aus Kohlenwasserstoffen mit höchstens zwei Kohlenstoffatomen bestehenden und Ethylen umfassenden Strom C2- aufgetrennt wird.

10. Verwendung einer Anlage, umfassend:
- einen Reaktor zur heterogen-katalysierten Umsetzung von Methanol und/oder Dimethylether (DME) als Oxygenate zu einem Produktstrom, der Wasser, Kohlenwasserstoffe und wenigstens ein Oxygenat enthält,
- wenigstens eine Quenchstufe zum Quenchen des Produktstroms, wodurch ein gasförmige Kohlenwasserstoffe und Olefine umfassender Strom G, ein flüssiger, zum überwiegenden Teil aus Wasser und Oxygenaten bestehender wässriger Strom W und ein flüssiger, zum überwiegenden Teil aus Kohlenwasserstoffen bestehender und Olefine umfassender Strom O erhalten wird,
- eine Trennvorrichtung zum Auftrennen des zum überwiegenden Teil aus Kohlenwasserstoffen bestehenden Stroms O in einen Strom C4-, welcher zum überwiegenden Teil aus Kohlenwasserstoffen mit vier oder weniger Kohlenstoffatomen besteht, und einen Strom C4+, welcher zum überwiegenden Teil aus Kohlenwasserstoffen mit vier oder mehr Kohlenstoffatomen besteht,
- **gekennzeichnet durch** eine Benzin-Stabilisatorkolonne und wenigstens eine Rückführleitung, mit der mindestens ein Teil des Stroms C4+ von der Benzin-Stabilisatorkolonne zum Reaktor zurückgeführt wird und/oder eine Leitung, durch die zumindest ein Teil des Stroms C4+ von der Benzin-Stabilisatorkolonne in eine Benzinfraktion geleitet wird,
in einem Verfahren zur Herstellung kurzkettiger Olefine gemäss Anspruch 1.

11. Verwendung einer Anlage gemäß Anspruch 10 zur Herstellung kurzkettiger Olefine aus Oxygenaten, wobei die Quenchstufe eine weitere Leitung umfasst, mit der ein weiterer flüssiger, zum überwiegenden Teil aus Kohlenwasserstoffen mit vier oder mehr Kohlenstoffatomen bestehender Strom S aus der Quenchstufe ausgeleitet wird.

12. Verwendung einer Anlage gemäß Anspruch 11 zur Herstellung kurzkettiger Olefine aus Oxygenaten, wobei die weitere Leitung so ausgestaltet ist, dass der Strom S mit dem Strom C4+ zusammengeführt wird und/oder zumindest ein Teil des Stroms S in die Benzinfraktion geleitet wird.

13. Verwendung einer Anlage gemäß Anspruch 10 oder 11 zur Herstellung kurzkettiger Olefine aus Oxygenaten, wobei Mittel umfasst werden, die es gestatten, dass Strom G und/oder Strom O einem bevorzugt mehrstufigen Kompressionsschritt zugeführt werden und aus diesem Kompressionsschritt, bevorzugt nach der ersten Stufe, ein zum überwiegenden Teil aus Kohlenwasserstoffen mit 4 oder mehr Kohlenstoffatomen bestehender Strom K erhalten wird.

14. Verwendung einer Anlage gemäß Anspruch 13 zur Herstellung kurzkettiger Olefine aus Oxygenaten, wobei Mittel umfasst werden, die es gestatten, dass der Strom K dem Strom S und/oder dem Strom C4+ zugemischt wird und/oder zumindest ein Teil des Stroms K direkt in die Benzinfraktion geleitet wird.

15. Verwendung einer Anlage gemäß Anspruch 13 zur Herstellung kurzkettiger Olefine aus Oxygenaten, wobei Mittel umfasst werden, die es gestatten, dass in dem Kompressionsschritt eine weitere flüssige Phase L erhalten wird, welche in die Trennvorrichtung eingeleitet wird.

16. Verwendung einer Anlage gemäß eines der Ansprüche 10 bis 15 zur Herstellung kurzkettiger Olefine aus Oxygenaten, wobei Mittel umfasst werden, die es gestatten, dass der Strom C4+ mindestens teilweise verdampft und der entstehende dampfförmige Strom D in Schritt (i) zurückgeführt wird.

17. Verwendung einer Anlage gemäß Anspruch 16 zur Herstellung kurzkettiger Olefine aus Oxygenaten, wobei Mittel umfasst werden, die es gestatten, dass der durch das teilweise Verdampfen im flüssigen Zustand verbleibende Strom F zumindest teilweise dem Strom S und/oder dem Strom K und/oder dem Strom C4+ zugemischt und/oder zumindest ein Teil direkt in die Benzinfraktion geleitet wird.

18. Verwendung einer Anlage gemäß eines der Ansprüche 10 bis 17 zur Herstellung kurzkettiger Olefine aus Oxygenaten, wobei die Trennvorrichtung in Fluidverbindung mit einer weiteren Aufreinigungsvorrichtung steht, wobei letztere Mittel umfasst, die es gestatten, der Strom C4- in einen zum überwiegenden Teil aus Kohlenwasserstoffen mit drei Kohlenstoffatomen bestehenden und Propylen umfassenden Strom C3 und in einen zum überwiegenden Teil aus Kohlenwasserstoffen mit höchstens zwei Kohlenstoffatomen bestehenden und Ethen umfassenden Strom C2- aufgetrennt wird.

## Claims

1. Process for preparing olefins from methanol and/or dimethyl ether (DME), comprising the following steps:
(i) heterogeneously catalysed conversion of the methanol and/or dimethyl ether as oxygenates in an oxygenate-to-olefin reactor under oxygenate conversion conditions to a product stream containing water, hydrocarbons and at least one oxygenate,
(ii) quenching the product stream in at least one quench stage to obtain a gaseous stream G comprising hydrocarbons and olefins, a liquid aqueous stream W consisting predominantly of water and oxygenates, and a liquid stream 0 consisting predominantly of hydrocarbons and comprising olefins,
(iii) separating stream G and/or stream 0 into a C4-stream consisting predominantly of hydrocarbons having four or fewer carbon atoms and comprising olefins, and a C4+ stream consisting predominantly of hydrocarbons having four or more carbon atoms and comprising olefins, **characterized in that** the C4+ stream obtained in step (iii) is returned directly to step (i) via a gasoline stabilizer column and/or guided into a gasoline fraction.

2. Process according to Claim 1, **characterized in that** a further liquid stream S consisting predominantly of hydrocarbons having four or more carbon atoms is obtained in step (ii).

3. Process according to Claim 2, **characterized in that** stream S is combined with the C4+ stream and/or at least a portion of stream S is guided into the gasoline fraction.

4. Process according to any of the preceding claims, **characterized in that** stream G and/or stream 0 is sent to a preferably multistage compression step and a liquid stream K consisting predominantly of hydrocarbons having 4 or more carbon atoms is obtained from this compression step, preferably after the first stage.

5. Process according to Claim 4, **characterized in that** stream K is mixed into stream S and/or the C4+ stream and/or at least a portion of stream K is guided directly into the gasoline fraction.

6. Process according to Claim 4, **characterized in that** a further liquid phase L is obtained in the compression step and introduced into step (iii).

7. Process according to any of the preceding claims, **characterized in that** the C4+ stream is at least partly evaporated and the resultant vaporous stream D is returned to step (i).

8. Process according to Claim 7, **characterized in that** the partial evaporating also gives a stream F that remains in the liquid state and is mixed at least partly with stream S and/or stream K and/or the C4+ stream and/or at least a portion is guided directly into the gasoline fraction.

9. Process according to any of the preceding claims, **characterized in that** step (iii) is followed by at least one further purification step in which the C4- stream is separated into a C3 stream consisting predominantly of hydrocarbons having three carbon atoms and comprising propylene and into a C2- stream consisting predominantly of hydrocarbons having not more than two carbon atoms and comprising ethylene.

10. Use of a plant comprising:
- a reactor for heterogeneously catalysed conversion of methanol and/or dimethyl ether (DME) as oxygenates to a product stream containing water, hydrocarbons and at least one oxygenate,
- at least one quench stage for quenching the product stream to obtain a gaseous stream G comprising hydrocarbons and olefins, a liquid aqueous stream W consisting predominantly of water and oxygenates, and a liquid stream 0 consisting predominantly of hydrocarbons and comprising olefins,
- a separation apparatus for separating stream 0 consisting predominantly of hydrocarbons into a C4-stream consisting predominantly of hydrocarbons having four or fewer carbon atoms and a C4+ stream consisting predominantly of hydrocarbons having four or more carbon atoms,
- **characterized by** a gasoline stabilizer column and at least one recycle conduit with which at least a portion of the C4+ stream from the gasoline stabilizer column is returned to the reactor and/or a conduit by which at least a portion of the C4+ stream is guided from the gasoline stabilizer column into a gasoline fraction, in a process for preparing short-chain olefins according to Claim 1.

11. Use of a plant according to Claim 10 for preparation of short-chain olefins from oxygenates, wherein the quench stage comprises a further conduit with which a further liquid stream S consisting predominantly of hydrocarbons having four or more carbon atoms is discharged from the quench stage.

12. Use of a plant according to Claim 11 for preparation of short-chain olefins from oxygenates, wherein the further conduit is configured such that stream S is combined with the C4+ stream and/or at least a portion of stream S is guided into the gasoline fraction.

13. Use of a plant according to Claim 10 or 11 for preparation of short-chain olefins from oxygenates, including means that permit sending of stream G and/or stream 0 to a preferably multistage compression step and obtaining of a stream K consisting predominantly of hydrocarbons having 4 or more carbon atoms from this compression step, preferably after the first stage.

14. Use of a plant according to Claim 13 for preparation of short-chain olefins from oxygenates, including means that permit mixing of stream K into stream S and/or the C4+ stream and/or guiding of at least a portion of stream K directly into the gasoline fraction.

15. Use of a plant according to Claim 13 for preparation of short-chain olefins from oxygenates, including means that permit obtaining of a further liquid phase L in the compression step which is introduced into the separation apparatus.

16. Use of a plant according to any of Claims 10 to 15 for preparation of short-chain olefins from oxygenates, including means that permit at least partial evaporating of the C4+ stream and returning of the resultant vaporous stream D into step (i).

17. Use of a plant according to Claim 16 for preparation of short-chain olefins from oxygenates, including means that permit at least partial mixing of stream F that remains in the liquid state via the partial evaporating with stream S and/or stream K and/or the C4+ stream and/or guiding of at least a portion directly into the gasoline fraction.

18. Use of a plant according to any of Claims 10 to 17 for preparation of short-chain olefins from oxygenates, wherein the separation apparatus is in fluid connection with a further purifying apparatus, the latter including means that permit separating of the C4- stream into a C3 stream consisting predominantly of hydrocarbons having three carbon atoms and comprising propylene and into a C2- stream consisting predominantly of hydrocarbons having not more than two carbon atoms and comprising ethene.

## Revendications

1. Procédé de préparation d'oléfines à partir de méthanol et/ou de diméthyléther (DME), comprenant les étapes suivantes :
(i) transformation catalysée par voie hétérogène du méthanol et/ou du diméthyléther en tant que composés oxygénés dans un réacteur composé oxygéné-à-oléfine dans des conditions de conversion d'oxygène en un flux produit, qui contient de l'eau, des hydrocarbures et au moins un composé oxygéné,
(ii) refroidissement brusque du flux produit dans au moins une étape de refroidissement brusque, suite à quoi on obtient un flux G gazeux comprenant des hydrocarbures et des oléfines, un flux W liquide, aqueux, constitué principalement d'eau et de composés oxygénés et un flux 0 liquide, constitué principalement d'hydrocarbures et comprenant des oléfines,
(iii) séparation du flux G et/ou du flux 0 en un flux C4-, qui est constitué principalement d'hydrocarbures comprenant quatre atomes de carbone ou moins et qui comprend des oléfines et en un flux C4+, qui est constitué principalement d'hydrocarbures comprenant quatre atomes de carbone ou plus et qui comprend des oléfines,
**caractérisé en ce que** le flux C4+ obtenu dans l'étape (iii) est recyclé directement par l'intermédiaire d'une colonne de stabilisation d'essence vers l'étape (i) et/ou guidé dans une fraction d'essence.

2. Procédé selon la revendication 1, **caractérisé en ce qu'**un autre flux S liquide, constitué principalement d'hydrocarbures comprenant quatre atomes de carbone ou plus est obtenu dans l'étape (ii).

3. Procédé selon la revendication 2, **caractérisé en ce que** le flux S est rassemblé avec le flux C4+ et/ou au moins une partie du flux S est guidée dans la fraction d'essence.

4. Procédé selon l'une des revendications précédentes, **caractérisé en ce que** le flux G et/ou le flux 0 est/sont introduit(s) dans une étape de compression de préférence à plusieurs étages et on obtient à partir de cette étape de compression, de préférence après le premier étage, un flux K liquide, constitué principalement d'hydrocarbures comprenant 4 atomes de carbone ou plus.

5. Procédé selon la revendication 4, **caractérisé en ce que** le flux K est mélangé au flux S et/ou au flux C4+ et/ou au moins une partie du flux K est guidée directement dans la fraction d'essence.

6. Procédé selon la revendication 4, **caractérisé en ce qu'**une autre phase L liquide, qui est introduite dans l'étape (iii), est obtenue dans l'étape de compression.

7. Procédé selon l'une des revendications précédentes, **caractérisé en ce que** le flux C4+ est au moins partiellement évaporé et le flux D sous forme vapeur formé est recyclé dans l'étape (i).

8. Procédé selon la revendication 7, **caractérisé en ce que**, un flux F restant à l'état liquide par l'évaporation partielle est également obtenu et mélangé au moins partiellement au flux S et/ou au flux K et/ou au flux C4+ et/ou au moins une partie est guidée directement dans la fraction d'essence.

9. Procédé selon l'une des revendications précédentes, **caractérisé en ce que** l'étape (iii) est suivie d'au moins une étape de purification, dans laquelle le flux C4- est séparé en un flux C3 constitué principalement d'hydrocarbures comprenant trois atomes de carbone et comprenant du propylène et en un flux C2- constitué principalement d'hydrocarbures comprenant au plus deux atomes de carbone et comprenant de l'éthylène.

10. Utilisation d'une installation, comprenant
- un réacteur destiné à la transformation catalysée par voie hétérogène de méthanol et/ou de diméthyléther (DME) en tant composés oxygénés en un flux produit, qui contient de l'eau, des hydrocarbures et au moins un composé oxygéné,
(ii) au moins une étape de refroidissement brusque destinée au refroidissement brusque du flux produit, suite à quoi on obtient un flux G gazeux comprenant des hydrocarbures et des oléfines, un flux W liquide, aqueux, constitué principalement d'eau et de composés oxygénés et un flux 0 liquide, constitué principalement d'hydrocarbures et comprenant des oléfines,
- un dispositif de séparation destiné à séparer le flux 0 constitué principalement d'hydrocarbures en un flux C4-, qui est constitué principalement d'hydrocarbures comprenant quatre atomes de carbone ou moins, et en une flux C4+, qui est constitué principalement d'hydrocarbures comprenant quatre atomes de carbone ou plus,
- **caractérisé par** une colonne de stabilisation d'essence et au moins une conduite de recyclage, par laquelle au moins une partie du flux C4+ est recyclée à partir de la colonne de stabilisation d'essence vers le réacteur et/ou une conduite, à travers laquelle au moins une partie du flux C4+ est guidée à partir de la colonne de stabilisation d'essence dans une fraction d'essence, dans un procédé de préparation d'oléfines à courte chaîne selon la revendication 1.

11. Utilisation d'une installation selon la revendication 10 pour la préparation d'oléfines à courte chaîne à partir de composés oxygénés, l'étape de refroidissement brusque comprenant une autre conduite qui permet d'évacuer un autre flux S liquide, constitué principalement d'hydrocarbures comprenant quatre atomes de carbone ou plus de l'étage de refroidissement brusque.

12. Utilisation d'une installation selon la revendication 11 pour la préparation d'oléfines à courte chaîne à partir de composés oxygénés, l'autre conduite étant conçue de telle sorte que le flux S est rassemblé avec le flux C4+ et/ou au moins une partie du flux S est guidée dans la fraction d'essence.

13. Utilisation d'une installation selon la revendication 10 ou 11 pour la préparation d'oléfines à courte chaîne à partir de composés oxygénés, des moyens étant compris, qui permettent que le flux G et/ou le flux 0 soi(en)t introduit(s) dans une étape de compression de préférence à plusieurs étages et un flux K constitué principalement d'hydrocarbures comprenant 4 atomes de carbone ou plus soit obtenu à partir de cette étape de compression, de préférence après le premier étage.

14. Utilisation d'une installation selon la revendication 13 pour la préparation d'oléfines à courte chaîne à partir de composés oxygénés, des moyens étant compris qui permettent que le flux K soit rassemblé avec le flux S et/ou le flux C4+ et/ou au moins une partie du flux K soit guidée directement dans la fraction d'essence.

15. Utilisation d'une installation selon la revendication 13 pour la préparation d'oléfines à courte chaîne à partir de composés oxygénés, des moyens étant compris qui permettent qu'une autre phase L liquide, qui est guidée dans le dispositif de séparation, soit obtenue dans l'étape de compression.

16. Utilisation d'une installation selon l'une des revendications 10 à 15 pour la préparation d'oléfines à courte chaîne à partir de produits oxygénés, des moyens étant compris qui permettent que le flux C4+ soit au moins partiellement évaporé et le flux D sous forme vapeur qui se forme soit recyclé dans l'étape (i).

17. Utilisation d'une installation selon la revendication 16 pour la préparation d'oléfines à courte chaîne à partir de composés oxygénés, des moyens étant compris qui permettent que le flux F restant à l'état liquide par l'évaporation partielle soit mélangé au moins partiellement au flux S et/ou au flux K et/ou au flux C4+ et/ou au moins une partie soit guidée directement dans la fraction d'essence.

18. Utilisation d'une installation selon l'une des revendications 10 à 17 pour la préparation d'oléfines à courte chaîne à partir de composés oxygénés, le dispositif de séparation étant en communication fluidique avec un autre dispositif de purification, ce dernier comprenant des moyens qui permettent que le flux C4- soit séparé en un flux C3 constitué principalement d'hydrocarbures comprenant trois atomes de carbone et comprenant du propylène et en un flux C2- constitué principalement d'hydrocarbures comprenant au plus deux atomes de carbone et comprenant de l'éthylène.
